# EUROPÄISCHE PATENTANMELDUNG

(11) **EP 2 258 830 A1**
(43) Veröffentlichungstag der Anmeldung: **08.12.2010**
(21) Anmeldenummer: 09007646.4
(22) Anmeldetag: 10.06.2009
(51) Int. Cl.: C12M 1/107

(54) **Fermentationsbehälter**

(30) Priorität: 04.06.2009 EP 09007400
(71) Anmelder: KOMPOFERM GmbH, 33428 Marienfeld (DE)
(72) Erfinder: Eggersmann, Karlgünter, 33428 Marienfeld (DE)
(74) Vertreter: Schober, Mirko

(57) **Zusammenfassung**

Ein Fermentationsbehälter (1) weist einen Innenraum (3) zur Beschickung mit einem Substrat(2) auf. Der Fermentationsbehälter (1) ist fluiddicht verschließbar ausgebildet. Er weist eine Einrichtung auf, welche dazu ausgelegt ist, das Volumen des Innenraumes (3) des Fermentationsbehälters (1) zu variieren.

## Beschreibung

Die Erfindung betrifft einen Fermentationsbehälter, der üblicherweise in Fermentationsprozessen zur Gewinnung von Biogas eingesetzt wird.

Entsprechende Behälter werden üblicherweise als Boxenfermenter mit einem Substrat beschickt und anschließend gasdicht verschlossen, bevor der Fermentationsprozess gestartet wird. Beim Fermentationsprozess entsteht methanhaltiges Biogas, welches aus dem Fermenter ab- und der weiteren Verwendung zugeführt wird. Nach dem Fermentationsprozess wird der Fermenter geöffnet und dabei zwangsläufig mit stickstoffhaltiger Frischluft gefüllt, während der Fermenter mit frischem Fermentationssubstrat für einen weiteren Fermentationsprozess befüllt wird. Auch bei Spülprozessen kann Frischluft in den Fermenter gelangen.

Bei der Biogasgewinnung sind die im Fermentervolumen vorhandenen Luftbestandteile (Stickstoff bzw. Sauerstoff) störend und deshalb unerwünscht.

Der Erfindung liegt daher die Aufgabe zugrunde, einen Fermenter anzugeben, bei dem dieser Nachteil nicht auftritt.

Gelöst wird diese Aufgabe mit den Merkmalen des Anspruchs 1. Vorteilhafte Ausführungsformen finden sich in den abhängigen Ansprüchen.

Erfindungsgemäß ist ein Fermentationsbehälter mit veränderlichem Volumen vorgesehen. Im einfachsten Fall kann dies erreicht werden durch wenigstens eine verschiebliche Innen-oder Außenwand. Bevorzugt ist eine Vorrichtung vorgesehen, welche durch Befüllen mit einem Fluid ihr Volumen ändert. Das Fluid ist im Regelfall ein Gas, kann aber auch eine Flüssigkeit sein. Nach einer bevorzugten Ausführungsform ist im Innern des Fermentationsbehälters eine fluiddichte Membran vorgesehen, wobei zwischen dieser und einer Fermenterwand ein mit Fluid befüllbarer Zwischenraum vorgesehen ist, in den das Fluid eingefüllt und aus welchem das Fluid wieder abgeführt werden kann. Die Membran ist zweckmäßigerweise flexibel oder elastisch ausgebildet, so dass sie im Falle der Befüllung des Zwischenraumes ihre Lage verändern oder ausdehnen und damit den Zwischenraum vergrößern und das Innenvolumen des Fermentationsbehälters, in welchem Prozessgas entsteht, entsprechend verkleinern kann. Wird der Fermentationsbehälter in dieser Situation belüftet, so steht gegenüber vom Substrat eingenommenen Volumen ein vergleichsweise geringes Volumen für Frischluft zur Verfügung. Damit kann in dieser Situation der Stickstoff- oder Sauerstoffgehalt im Verhältnis zur Biomasse gegenüber bekannten Fermentern verringert werden. Wesentlich hierfür ist, dass das Volumen des Innenraumes, in welchem sich auch das Substrat befindet, vor dem eigentlichen Start des Fermentationsprozesses durch geeignete Maßnahmen reduziert wird. Dabei kann die Volumenreduzierung vor oder nach dem durch Öffnen des Behälters oder durch gezieltes Spülen desselben erfolgenden Eintrag von Stickstoff bzw. Sauerstoff in den Fermentationsbehälter vorgenommen werden.

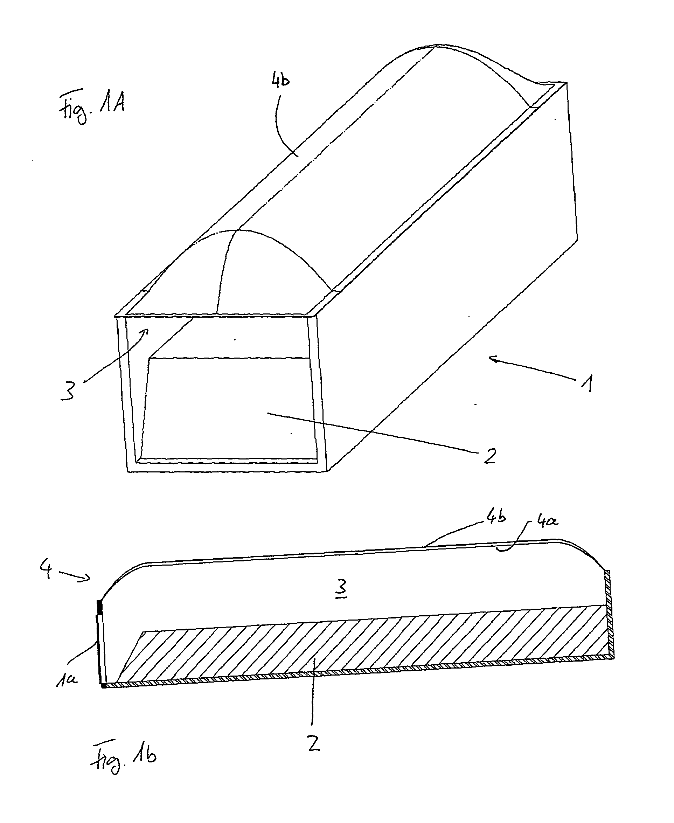

Die Erfindung wird nachfolgend anhand einer bevorzugten Ausführungsform in den Figuren 1A bis 2C schematisch näher erläutert.

| | |
|---|---|
| Figuren 1A bis 1C - | zeigen einen erfindungsgemäßen Fermentationsbehälter in perspektivischer Ansicht (Fig. 1A) sowie als Längsschnitt (Fig. 1B) und Querschnitt (Fig. 1C) in einem ersten Betriebszustand mit großem Innenvolumen, |
| Figuren 2A bis 2C - | zeigen einen erfindungsgemäßen Fermentationsbehälter in perspektivischer Ansicht (Fig. 2A) sowie als Längsschnitt (Fig. 2B) und Querschnitt (Fig. 2C) in einem zweiten Betriebszustand mit reduziertem Innenvolumen. |

Der in den Figuren 1A bis 1C gezeigte Fermentationsbehälter 1 (im Folgenden kurz Fermenter genannt) weist einen durch Seitenwände begrenzten Innenraum 3 auf. Dieser lässt sich fluiddicht verschließen, wobei der Fermenter 1 bevorzugt eine Klappe 1a aufweist, durch welche der Innenraum 3 für die Beschickung mit Fermentationsmaterial 2 (Substrat) zugänglich ist. Infolge technischer Grenzen - zumeist wird das Substrat 2 mit Radladern in den Innenraum 3 verbracht, kann der Innenraum 3 nicht vollständig (d.h. bis unter das Dach 4b) mit Substrat 2 aufgefüllt werden und es verbleibt nach der Beschickung immer ein freies Restvolumen. Zudem ist ein bestimmtes Restvolumen erforderlich, um das bei der Fermentation entstehende Biogas aufzufangen und über entsprechende Anschlüsse in der Fermenterwand abzuführen.

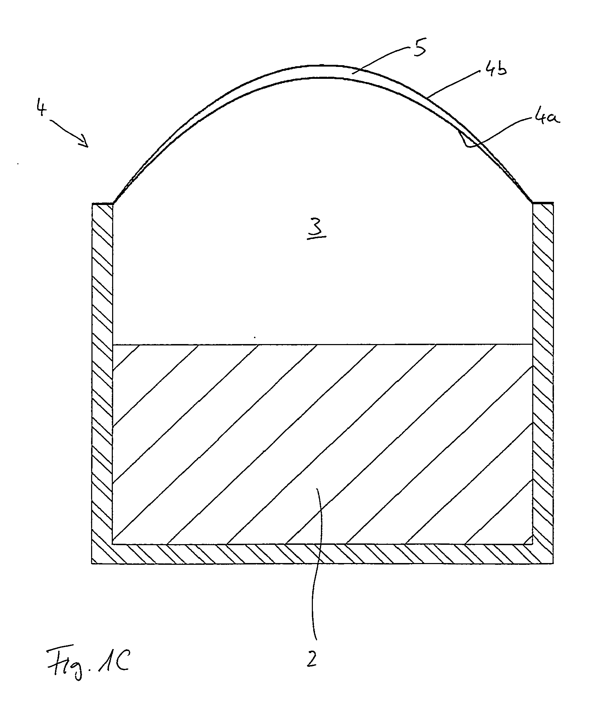

Die Erfindung weist nun eine - im gezeigten Beispiel aus dem Fermenterdach 4b und einer als Membran ausgebildeten fluiddichten Trennwand 4a gebildete - Vorrichtung auf, mit der das Volumen 3 (zwischen Substrat 2 und den Fermenterinnenwänden) verringert werden kann. Die Membran 4a ist flexibel ausgebildet und weist gegenüber der Innenseite des Daches 4b, welches bevorzugt gewölbt ausgebildet ist, eine etwas geringere Oberfläche auf, so dass (vgl. Fig. 1C) zwischen Trennwand 4a und dem bevorzugt aus einem starren Material gebildeten Dach 4b ein mit einem Fluid (Gas oder Flüssigkeit) befüllbarer Zwischenraum 5 entsteht.

Soll nun im gezeigten Beispiel das Volumen 3 verringert werden, wird der Zwischenraum 5 mit einem Fluid gefüllt, wodurch sich der Zwischenraum 5 von dem in Figur 1C gezeigten Zustand infolge der Lageänderung oder Elastizität der Membran 4a vergrößert.

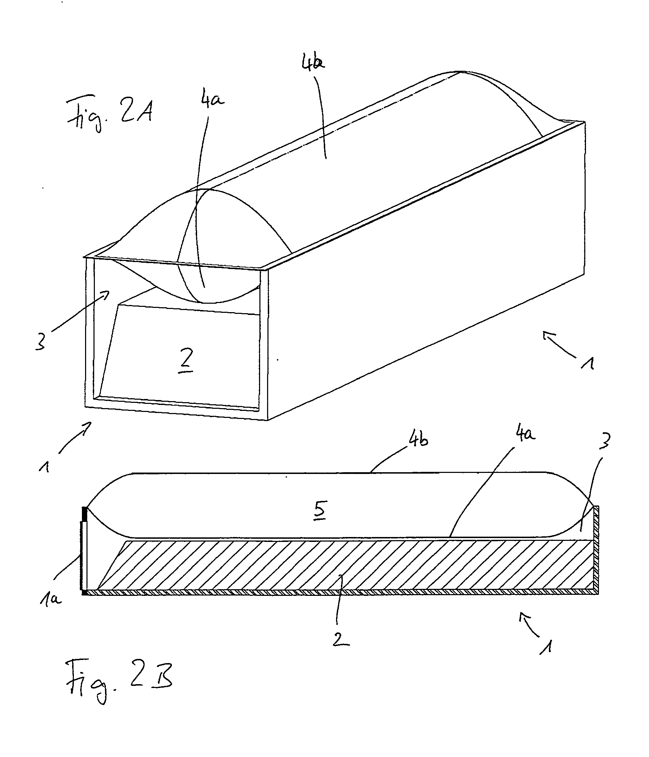

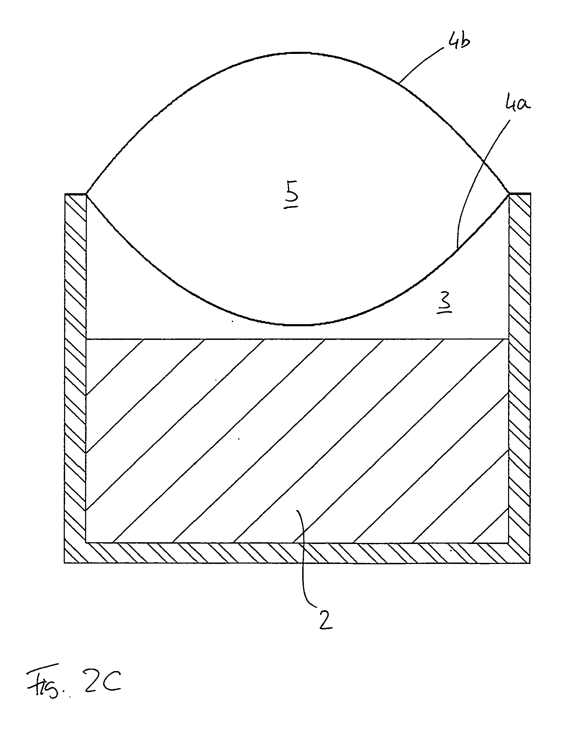

Diese Situation ist in den Figuren 2A bis 2C gezeigt. Wie man insbesondere aus der Querschnittzeichnung der Figur 1C erkennt, ist das verbleibende Volumen 3 durch diese Maßnahme stark verringert. Erfolgt nun ein Stickstoffeintrag (z.B. über Frischluft) in den Fermenter 1, so kann im Vergleich zum vom Substrat 2 eingenommenen Volumen lediglich ein geringer Volumenanteil 3 (Fig. 1C) mit Stickstoff befüllt werden.

Die Vorrichtung 4, d.h. die Membran 4a, kann auch zum Austreiben stickstoffhaltiger Gase verwendet werden. Ist zum Beispiel beim Beschicken mit Substrat 2 die Klappe 1a geöffnet, und befindet sich die Membran 4a in der in Figur 1A bis 1C gezeigten Lage, so kann der Zwischenraum 5 nach Schließen der Klappe 1a durch Befüllen vergrößert werden. Bei gleichzeitigem Öffnen eines Gasauslasses (nicht gezeigt) wird so ein Teil des stickstoffhaltigen Gases infolge der oben beschriebenen Volumenverringerung aus dem Innenraum 3 heraus gedrängt.

Die Erfindung ist nicht auf die gezeigte Ausführungsform beschränkt. Andere Möglichkeiten zur Volumenänderung wie z.B. Ballons im Innern des Fermenters oder ein absenkbares Dach wären ebenso denkbar.

## Patentansprüche

1. Fermentationsbehälter (1) mit einem Innenraum (3) zur Beschickung mit einem Substrat(2), welcher fluiddicht verschließbar ausgebildet ist und eine Einrichtung aufweist, welche dazu ausgelegt ist, das Volumen des Innenraumes (3) des Fermentationsbehälters (1) zu variieren.

2. Fermentationsbehälter (1) nach Anspruch 1,
**dadurch gekennzeichnet,**
**dass** die Vorrichtung (4) über wenigstens eine Trennwand (4a) vom übrigen Volumen des Innenraumes (3) fluiddicht getrennt und mit Fluid befüllbar ist.

3. Fermentationsbehälter (1) nach Anspruch 2,
**dadurch gekennzeichnet,**
**dass** die befüllbare Vorrichtung aus einer im Wesentlichen festen ersten Wand (4b) und wenigstens der Trennwand (4a) gebildet ist, wobei zwischen der ersten Wand (4b) und der Trennwand (4a) ein mit Fluid befüllbares Volumen (5) gebildet ist.

4. Fermentationsbehälter (1) nach Anspruch 3,
**dadurch gekennzeichnet,**
**dass** die erste Wand (4b) eine Außenwand des Fermentationsbehälters (1) ist.

5. Fermentationsbehälter (1) nach einem der Ansprüche 3 oder 4,
**dadurch gekennzeichnet, dass** die erste Wand (4b) gewölbt ist.

6. Fermentationsbehälter (1) nach einem der Ansprüche 3 bis 5,
**dadurch gekennzeichnet,**
**dass** die Innenfläche der ersten Wand (4b) größer ist als die Oberfläche der Trennwand (4a).

7. Fermentationsbehälter (1) nach einem der vorherigen Ansprüche,
**dadurch gekennzeichnet,**
**dass** die Trennwand (4a) als Membran ausgebildet ist.

8. Verfahren zum Betreiben einer Fermentationsanlage mit einem Fermentationsbehälter (1), insbesondere nach einem der vorherigen Ansprüche, bei welchem der Innenraum (3) des Fermentationsbehälters (1) mit Substrat beschickt wird und wobei der Innenraum (3) zudem mit einem stickstoffhaltigen und/oder sauerstoffhaltigen Gas, insbesondere Frischluft, durch Öffnen des Fermentationsbehälters (1) oder durch Spülen desselben befüllt wird,
**dadurch gekennzeichnet,**
**dass** das Volumen des Innenraums (3) des Fermentationsbehälters (1) vor und/oder nach dem Befüllen mit stickstoff- und/oder sauerstoffhaltigem Gas verringert wird.
